# EUROPEAN PATENT APPLICATION

(11) **EP 4 502 160 A1**
(43) Date of publication of application: **05.02.2025**
(21) Application number: 23775121.9
(22) Date of filing: 24.03.2023
(51) Int. Cl.: C12N 15/54, C12N 1/15, C12N 1/19, C12N 1/21, C12N 5/10, C12N 9/10, C12P 19/04

(54) **PROTEIN HAVING ?1,2-FUCOSYLTRANSFERASE ACTIVITY AND METHOD FOR PRODUCING LACTO-N-FUCOPENTAOSE I (LNFPI)**

(30) Priority: 25.03.2022 JP 2022050798
(71) Applicant: KIRIN HOLDINGS KABUSHIKI KAISHA, Nakano-ku, Tokyo 164-0001 (JP)
(72) Inventor: KAMAI Sayaka, Tokyo 164-0001 (JP); SUGITA Tomotoshi, Tokyo 164-0001 (JP); YAMAZAKI Fuhito, Tokyo 164-0001 (JP); NAKAMURA Kazuki, Tokyo 164-0001 (JP)
(74) Representative: Grünecker Patent- und Rechtsanwälte PartG mbB
(86) International application number: PCT/JP2023/012042
(87) International publication number: WO 2023/182528

(57) **Abstract**

The purpose of the present invention is to provide: a protein that has an α1,2-fucosyltransferase activity and that has excellent LNFPI productivity; and a method for producing LNFPI. The present invention pertains to: a protein that has an activity of transferring fucosyl groups to lacto-N-tetraose (LNT) and that has an amino acid sequence represented by SEQ ID NO. 2, 4, 6, 8, 10, 14, 18, or 26; or a mutant protein or a homologous protein thereof.

## Description

### TECHNICAL FIELD

The present invention relates to a protein having an α1,2-fucosyltransferase activity and a method for producing lacto-N-fucopentaose I (LNFPI).

### BACKGROUND ART

Human milk oligosaccharides (HMO) have attracted attention as prebiotics materials, and have been shown to be effective in development of cognitive functions, infection protection, and improvement of intestinal environment in infants (Non Patent Literature 1).

Lacto-N-fucopentaose I (hereinafter, referred to as LNFPI) is a type of HMO, and is a pentasaccharide HMO in which fucose is bonded to 2-position of galactose in lacto-N-tetraose (hereinafter, referred to as LNT) via an α1,2-bond.

LNFPI is contained in a large amount in human milk subsequent to 2'-fucosyllactose (hereinafter, referred to as 2'FL) and lacto-N-difucohexaose (hereinafter, referred to as LNDFHI), and is known to be present in a higher amount in human milk as compared with lacto-N-fucopentaose II (hereinafter, referred to as LNFPII) and lacto-N-fucopentaose III (hereinafter, referred to as LNFPIII), which are also pentasaccharides and are known to be isomers of LNFPI (Non Patent Literature 2).

The functionality of LNFPI is known to have an inhibitory effect against meningitis-causing group B Streptococcus (GBS) and a norovirus inhibitory effect (Non Patent Literatures 3 and 4). Bifidobacterium infantis, which has a high occupancy rate in the intestines of newborns, has been shown to grow preferentially in LNFPI-selective media, and as a result, a prebiotic function thereof is also attracting attention (Non Patent Literature 5).

As a method for producing LNFPI, a microbial fermentation method using α1,2-fucosyltransferase or an enzyme reaction method [One-pot multienzyme (OPME) system] is widely used. Patent Literatures 1 and 2 and Non Patent Literatures 4, 5, and 6 disclose a method for producing an oligosaccharide such as LNFPI by overexpressing α1,2-fucosyltransferase derived from a microorganism such as Thermosynechococcus elongatus, Sideroxydans lithotrophicus, or Helicobacter pylori in Escherichia coli and using LNT and GDP-fucose as substrates through a fermentation method or a continuous enzyme reaction method.

However, in the above-described fermentation method or continuous enzyme reaction method, a problem with LNFPI production is that α1,2-fucosyltransferase reacts not only with the desired substrate LNT but also with coexisting lactose to produce 2'FL as a by-product.

As a method for reducing a by-product, an enzyme reaction method using highly purified LNT as a substrate (Patent Literature 1, Non Patent Literature 5), and a method for producing LNFPI by inducing expression of α1,2-fucosyltransferase when an initial raw material lactose is depleted (Non-Patent Literature 6).

### CITATION LIST

### PATENT LITERATURE

Patent Literature 1: WO2017/106864
Patent Literature 2: WO2019/008133

### NON-PATENT LITERATURE

Non Patent Literature 1: Int. J. Pediatrics (2019), Article ID 2390240
Non Patent Literature 2: Nutr. Rev. (2017) 75, 920-933
Non Patent Literature 3: J. Biol. Chem. (2017) 292 (27) 11243-11249
Non Patent Literature 4: J. Biotechnol. (2020) 318, 31-38
Non Patent Literature 5: Chem. Commun. (2016) 52, 3899-3902
Non Patent Literature 6: Bioorganic & Medicinal Chemistry 23(2015) 6799-6806

### SUMMARY OF INVENTION

### TECHNICAL PROBLEM

As described above, there has been known a microbial fermentation method and an enzyme reaction method using α1,2-fucosyltransferase. However, since α1,2-fucosyltransferase derived from microorganisms described in Patent Literatures 1 and 2 and Non Patent Literatures 4, 5, and 6 can tolerate a wide range of saccharide substrates, 2'FL is produced as a by-product during LNFPI production.

On the other hand, in order to produce LNFPI more efficiently using lactose as an initial raw material, α1,2-fucosyltransferase that does not transfer saccharide to lactose but can selectively transfer saccharide to a non-reducing terminal galactose site of LNT is required.

Therefore, an object of the present invention is to provide a protein having an α1,2-fucosyltransferase activity and excellent productivity of LNFPI, and a method for producing LNFPI.

### SOLUTION TO PROBLEM

The present inventors have found that LNFPI can be efficiently produced by using a microorganism having an ability to produce a protein having an α1,2-fucosyltransferase activity and consisting of a specific amino acid sequence, as compared with a method in related art, and has completed the present invention.

The present inventors have found for the first time fucosyltransferase derived from the genus Neisseria or the genus Francisella that is suitable for production of fucosylated oligosaccharides such as LNFPI or fucosyl lactose.

That is, the present invention is as follows.
1. A protein according to any one of the following [1] to [3], which has a trans-fucosylation activity to lacto-N-tetraose (LNT):
   [1] a protein consisting of the amino acid sequence represented by SEQ ID NO: 2, 4, 6, 8, 10, 14, 18, or 26,
   [2] a mutant protein having an α1,2-fucosyltransferase activity and consisting of an amino acid sequence in which 1 to 20 amino acids are deleted, substituted, inserted, or added in the amino acid sequence represented by SEQ ID NO: 2, 4, 6, 8, 10, 14, 18, or 26, and
   [3] a homologous protein having an α1,2-fucosyltransferase activity and consisting of an amino acid sequence having an identity of 90% or more with the amino acid sequence represented by SEQ ID NO: 2, 4, 6, 8, 10, 14, 18, or 26.
2. A DNA consisting of the nucleotide sequence represented by SEQ ID NO: 1, 3, 5, 7, 9, 13, 17, or 25 or a homologous sequence thereof and encoding the protein according to any one of [1] to [3] according to the above 1.
3. A recombinant DNA comprising the DNA according to the above 2.
4. A transformant obtained by transforming a host cell with the recombinant DNA according to the above 3.
5. The transformant according to the above 4, which is a microorganism having an enhanced activity of the protein according to any one of [1] to [3] according to the above 1 and enhanced productivity of fucose-containing carbohydrate.
6. The transformant according to the above 5, in which the microorganism is Escherichia coli.
7. A method for producing a fucose-containing carbohydrate, including: preparing the transformant according to any one of the above 4 to 6; and producing the fucose-containing carbohydrate in a culture using the transformant.
8. The production method according to the above 7, in which the fucose-containing carbohydrate is lacto-N-fucopentaose I (LNFPI).

### ADVANTAGEOUS EFFECTS OF INVENTION

The protein of the present invention consists of a specific amino acid sequence and thus has an α1,2-fucosyltransferase activity capable of transferring a saccharide to a non-reducing terminal galactose site of LNT By using a microorganism having an ability to produce the protein of the present invention, production of byproducts can be prevented and LNFPI can be produced efficiently as compared with that in related art.

### BRIEF DESCRIPTION OF DRAWINGS

[FIG. 1] FIG. 1 shows a biosynthetic pathway of LNFPI in one embodiment of the present invention.
[FIG. 2] FIG. 2 shows results of combined amounts of LNFPI produced in a supernatant and an intracellular fraction (Example 2).

### DESCRIPTION OF EMBODIMENTS

### <Protein, DNA, and Transformant>

A protein of the present invention is a protein according to any one of the following [1] to [3], which has a trans-fucosylation activity to lacto-N-tetraose (LNT):
[1] a protein consisting of the amino acid sequence represented by SEQ ID NO: 2, 4, 6, 8, 10, 14, 18, or 26,
[2] a mutant protein having an α1,2-fucosyltransferase activity and consisting of an amino acid sequence in which 1 to 20 amino acids are deleted, substituted, inserted, or added in the amino acid sequence represented by SEQ ID NO: 2, 4, 6, 8, 10, 14, 18, or 26, and
[3] a homologous protein having an α1,2-fucosyltransferase activity and consisting of an amino acid sequence having an identity of 90% or more with the amino acid sequence represented by SEQ ID NO: 2, 4, 6, 8, 10, 14, 18, or 26.

From the viewpoint of further enhancing the trans-fucosylation activity to LNT, among the proteins according to the above [1], a protein consisting of the amino acid sequence represented by SEQ ID NO: 4, 6, 8, 14, 18, or 26 is preferred, and a protein consisting of the amino acid sequence represented by SEQ ID NO: 4 or 18 is more preferred.

The protein consisting of the amino acid sequence represented by SEQ ID NO: 2 is α1,2-fucosyltransferase GsFucT derived from the Gramella sp. MAR_2010_147 strain, which will be described later in Examples.

The protein consisting of the amino acid sequence represented by SEQ ID NO: 4 is α1,2-fucosyltransferase FsFucT derived from the Francisella sp. FSC1006 strain, which will be described later in Examples.

The protein consisting of the amino acid sequence represented by SEQ ID NO: 6 is α1,2-fucosyltransferase NbFucT1 derived from the Neisseriaceae bacterium DSM 100970 strain, which will be described later in Examples.

The protein consisting of the amino acid sequence represented by SEQ ID NO: 8 is α1,2-fucosyltransferase MtFucT derived from the Methylobacter tundripaludum strain, which will be described later in Examples.

The protein consisting of the amino acid sequence represented by SEQ ID NO: 10 is α1,2-fucosyltransferase AjFucT derived from the Amphritea japonica strain, which will be described later in Examples.

The protein consisting of the amino acid sequence represented by SEQ ID NO: 14 is α1,2-fucosyltransferase SbFucT derived from the Sterolibacteriaceae bacterium J5B strain, which will be described later in Examples.

The protein consisting of the amino acid sequence represented by SEQ ID NO: 18 is α1,2-fucosyltransferase NbFucT2 derived from the Neisseriales bacterium strain, which will be described later in Examples.

The protein consisting of the amino acid sequence represented by SEQ ID NO:26 is α1,2-fucosyltransferase HMFT derived from the Helicobacter mustelae ATCC 43772 strain, which will be described later in Examples.

In the present description, the mutant protein refers to a protein obtained by artificially deleting or substituting an amino acid residue of an original protein or inserting or adding an amino acid residue in the protein.

The expression "an amino acid is deleted, substituted, inserted, or added in the mutant protein of the above [2]" may mean that 1 to 20 amino acids are deleted, substituted, inserted, or added at any position in the same sequence. The number of amino acids to be deleted, substituted, inserted, or added is preferably 1 to 20, more preferably 1 to 10, further preferably 1 to 8, and most preferably 1 to 5.

The amino acid to be deleted, substituted, inserted, or added may be of a natural type or a non-natural type. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

In the mutant protein of the above [2], examples of the amino acid residue to be substituted include an asparagine residue at position 17.

In the present description, the homologous protein is a protein whose encoding gene is thought to have the same evolutionary origin as a gene encoding an original protein due to similarity in structure and function with the original protein, and is a protein possessed by organisms in nature.

Examples of the homologous protein include an amino acid sequence having an identity of preferably 90% or more, more preferably 93% or more, further preferably 95% or more, and particularly preferably 97% or more with the amino acid sequence of a target protein.

The identity of an amino acid sequence and a nucleotide sequence can be determined by using an algorithm BLAST [Pro. Natl. Acad. Sci. USA, 90, 5873 (1993)] or FASTA [Methods Enzymol., 183, 63 (1990)] developed by Karlin and Altschul. Programs called BLASTN and BLASTX have been developed based on the algorithm BLAST [J. Mol. Biol., 215, 403 (1990)]. When the nucleotide sequence is analyzed by BLASTN based on BLAST, the parameters are, for example, Score = 100 and wordlength = 12. When the amino acid sequence is analyzed by BLASTX based on BLAST, the parameters are, for example, score = 50 and wordlength = 3. When BLAST and Gapped BLAST programs are used, default parameters for each program are used. A specific method of the analysis methods is well known.

In the present description, the trans-fucosylation activity to LNT refers to an activity of transferring a fucose residue from GDP-fucose, which is a donor substrate, to a hydroxyl group of N-acetylglucosamine in LNT, which is a carbohydrate which is a receptor substrate (hereinafter, referred to as "receptor carbohydrate").

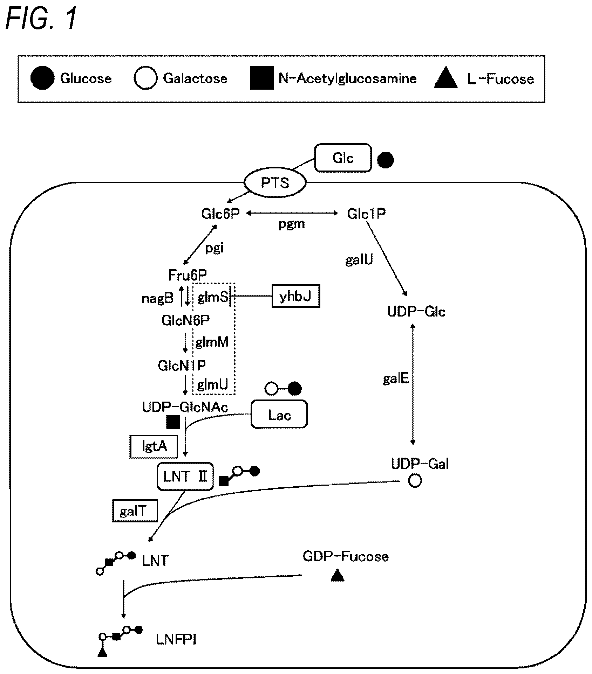

LNFPI is generated by transfer of a fucose residue from GDP-fucose to a hydroxyl group of N-acetylglucosamine. FIG. 1 shows a biosynthetic pathway of LNFPI in one embodiment of the present invention.

In the present description, the α1,2-fucosyltransferase activity refers to an activity of transferring a fucose residue from a donor substrate GDP-fucose to a hydroxyl group of N-acetylglucosamine in a receptor carbohydrate via an α1,2-bond to generate a fucose-containing carbohydrate. The receptor carbohydrate is preferably LNT. The fucose-containing carbohydrate is preferably LNFPI.

It can be confirmed by, for example, the following method that the above mutant protein or homologous protein has an α1,2-fucosyltransferase activity.

First, a recombinant DNA comprising a DNA encoding the above mutant protein or homologous protein whose activity is to be confirmed is prepared by a method to be described later. Next, a transformant having a higher activity of the protein as compared with a parent strain is prepared by transforming the parent strain with the recombinant DNA, and amounts of fucose-containing carbohydrates produced and accumulated in culture solutions of the parent strain and the transformant are compared to confirm. Specific examples of the fucose-containing carbohydrate include LNFPI.

In the present description, the term "parent strain" refers to an original strain to be subjected to genetic modification, transformation, and the like.

In the present description, the parent strain is preferably a prokaryote or a yeast strain, more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and most preferably a prokaryote such as Escherichia coli MG1655, Escherichia coli XL1 -Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli BL21 codon plus (manufactured by Stratagene Corporation), Escherichia coli W3110S3GK (NBRC114657), Serratia ficaria, Serratia fonticola, Serratia liquefaciens, Serratia marcescens, Bacillus subtilis, Bacillus amyloliquefaciens, Brevibacterium immariophilum ATCC 14068, Brevibacterium saccharolyticum ATCC 14066, Corynebacterium ammoniagenes, Corynebacterium glutamicum ATCC 13032, Corynebacterium glutamicum ATCC 14067, Corynebacterium glutamicum ATCC 13869, Corynebacterium acetoacidophilum ATCC 13870, Microbacterium ammoniaphilum ATCC 15354, or Pseudomonas sp. D-0110, or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

The parent strain may be a wild strain as long as it is a microorganism that produces GDP-fucose and/or LNT. When a wild strain does not have an ability to produce GDP-fucose and/or LNT, the wild strain may be a bred strain to which an ability to supply GDP-fucose and/or LNT is artificially endowed.

Examples of the microorganism that can be used as a parent strain include the following 1) and 2).
1) A microorganism having an artificially endowed or enhanced ability to supply GDP-fucose, which is a reaction substrate for α1,2-fucosyltransferase
2) A microorganism having an artificially endowed or enhanced ability to supply LNT, which is a reaction substrate for α1,2-fucosyltransferase

This will be described below.
1) A microorganism used as a parent strain and having an artificially endowed or enhanced ability to supply GDP-fucose, which is a reaction substrate for α1,2-fucosyltransferase

The parent strain is preferably a microorganism having an artificially endowed or enhanced ability to supply GDP-fucose, which is a reaction substrate for α1,2-fucosyltransferase. Specific examples of the method for endowing or enhancing an ability to supply GDP-fucose to a microorganism used as a parent strain include a known method such as a method using various genetic manipulations (Metabolic Engineering (2017) 41: 23-38).

Examples of the ability to supply GDP-fucose include an ability to produce GDP-fucose from a saccharide. Examples of the method for artificially endowing or enhancing an ability to supply GDP-fucose from a saccharide to a microorganism used as a parent strain include the following methods (1a) to (1d). These methods may be used alone or in combination.
(1a) A method of alleviating or releasing at least one mechanism for controlling a biosynthetic pathway for producing GDP-fucose from a saccharide
(1b) A method of enhancing expression of at least one enzyme associated with a biosynthetic pathway for producing GDP-fucose from a saccharide
(1c) A method of increasing the number of copies of at least one gene encoding an enzyme associated with a biosynthetic pathway for producing GDP-fucose from a saccharide
(1d) A method of weakening or blocking at least one metabolic pathway branching off from a biosynthetic pathway for producing GDP-fucose from a saccharide to a metabolic product other than a target substance

Specific examples of the mechanism for controlling a biosynthetic pathway for producing GDP-fucose from a saccharide include known mechanisms such as a control mechanism based on a transcription regulatory factor (e.g., RcsA) associated with control of the biosynthetic pathway. RcsA is a regulatory factor for uppreforming the entire cholanic acid biosynthetic pathway using GDP-fucose as an intermediate. As will be described later, by strengthening rcsA in a state where a pathway downstream of GDP-fucose in the cholanic acid biosynthetic pathway is blocked, a large amount of GDP-fucose can be accumulated.

Specific examples of the enzyme associated with the biosynthetic pathway for producing GDP-fucose from a saccharide include known enzymes such as a mannose-6-phosphate isomerase, a phosphomannomutase, a mannose-1-phosphate guanylyltransferase, a GDP mannose-4,6-dehydratase, and a GDP-L-fucose synthase.

Specific examples of the metabolic pathway branching off from a biosynthetic pathway for producing GDP-fucose from a saccharide to a metabolic product other than a target substance include a known metabolic pathway such as a metabolic pathway from GDP-fucose to cholanic acid. Particularly, the supply of GDP-fucose can be enhanced by blocking WcaJ, WzxC, WcaK, WcaL, or WcaM, which is a pathway downstream of GDP-fucose in the cholanic acid biosynthetic pathway.

The microorganism to be used as a parent strain may be modified to promote transfer of exogenous L-fucose crossing a cell membrane thereof. For example, by expressing or overexpressing the nucleotide sequence (Accession Number: AIZ90162) encoding FucP, uptake of exogenous L-fucose crossing the cell membrane into cells can be improved, and thus a fucose amount for producing GDP-fucose can be further increased.

The microorganism to be used as a parent strain may be modified such that genes fuel and/or fucK encoding L-fucose isomerase and L-fuculose kinase, respectively, are deleted, and nucleotide sequences of fuel and/or fucK are changed to irreversibly inactivate an enzyme activity of a corresponding polypeptide, or expression of fuel and/or fucK is impaired. When intracellular synthesis of Fuel and/or FucK is eliminated, fucose metabolism in cells disappears, thereby allowing for increased amounts of fucose to produce GDP-fucose.

2) A microorganism used as a parent strain and having an artificially endowed or enhanced ability to supply LNT, which is a reaction substrate for α1,2-fucosyltransferase
Examples of a method for artificially endowing the ability to supply LNT to a microorganism used as a parent strain include the following methods (2a) to (2h), and these methods can be used alone or in combination.
(2a) A method of alleviating or releasing at least one mechanism for controlling a biosynthetic pathway for producing LNT from a saccharide
(2b) A method of enhancing expression of at least one enzyme associated with the biosynthetic pathway for producing LNT from a saccharide
(2c) A method of increasing the number of copies of at least one enzyme gene associated with the biosynthetic pathway for producing LNT from a saccharide
(2d) A method of alleviating or releasing at least one mechanism for decomposing LNT or a saccharide that is a substrate thereof
(2e) A method of enhancing an expression of at least one enzyme associated with intracellular uptake of LNT or a saccharide that is a substrate thereof
(2f) A method of increasing the number of copies of at least one gene encoding an enzyme associated with intracellular uptake of LNT or a saccharide that is a substrate thereof
(2g) A method of weakening or blocking at least one metabolic pathway branching off from a biosynthetic pathway for producing LNT from a saccharide to a metabolic product other than a target substance
(2h) A method of selecting a cell strain having resistance to an LNT analogue higher than that of a wild strain

Specific examples of the enzyme associated with the biosynthetic pathway for producing LNT from a saccharide include known enzymes such as an enzyme associated with a biosynthetic pathway for producing LNT from glucose and lactose and having a β1,4-galactosyltransferase (hereinafter, referred to as galT) activity and an enzyme having a β1,3-N-acetylglucosaminetransferase (hereinafter, referred to as lgtA) activity.

Specific examples of the mechanism for decomposing LNT or a saccharide as a substrate thereof include known enzymes such as β-galactosidase that catalyze hydrolysis of lactose as a substrate for LNT to produce glucose and galactose. Specific examples thereof include β-galactosidase (hereinafter, referred to as lacZ) that hydrolyzes lactose as a substrate for LNT, and a decrease in supply of lactose can be prevented by losing the activity of lacZ.

Specific examples of the enzyme associated with intracellular uptake of LNT or a saccharide as a substrate thereof include known enzymes such as a lactose permease associated with intracellular uptake of lactose as a substrate for LNT.

Specifically, for example, in order to supply LNT, the microorganism having an endowed or enhanced ability to supply LNT preferably has at least one activity selected from a lactose permease (hereinafter, referred to as lacY) activity, a β1,4-galactosyltransferase (galT) activity, a β1,3-N-acetylglucosaminetransferase (lgtA) activity, a glutamine fructose-6-phosphate transaminase (hereinafter, referred to as glmS) activity, a phosphoglucosamine mutase (hereinafter, referred to as glmM) activity, a N-acetylglucosamine-1-phosphate uridyltransferase/glucosamine-1-phosphate acetyltransferase (hereinafter, referred to as glmU) activity, a phosphoglucomutase (hereinafter, referred to as pgm) activity, an UTP glucose-1-phosphate uridyltransferase (hereinafter, referred to as galU) activity, an UDP glucose-4-epimerase (hereinafter, referred to as galE) activity, an UTP glucose-1-phosphate uridyltransferase (hereinafter, referred to as galF) activity, a glucose-6-phosphate isomerase (hereinafter, referred to as pgi) activity, and more preferably has enhanced activities thereof.

Among them, the microorganism preferably has lacY, galT, and lgtA activities, and more preferable has enhanced activities thereof.

lacY is a membrane protein that takes up lactose, which is a substrate of LNT, into cells. galT is an enzyme associated with generation of LNT from lacto-N-triose II (LNTII). LNT is a precursor of LNFPI. LgtA is an enzyme associated with production of LNTII from lactose and uridine diphosphate-N-acetylglucosamine (hereinafter, referred to as UDP-GlcNAc). LNTII is a precursor of LNT.

glmS, glmM, and glmU are enzymes associated with a biosynthetic pathway for producing LNTII. Pgm, galU, galE, and galF are enzymes associated with a pathway for producing uridine diphosphate galactose (hereinafter, referred to as UDF-Gal). Pgi is an enzyme associated with a pathway for producing LNTII.

The fact that the microorganism is a microorganism capable of producing GDP-fucose and/or LNT can be confirmed by culturing the microorganism in a culture medium and detecting GDP-fucose and/or LNT accumulated in a culture by using a general method such as a carbohydrate analyzer or a high performance liquid chromatograph mass spectrometer to be described later.

The microorganism used as a parent strain of the present invention is preferably a microorganism having an artificially endowed or enhanced ability to supply GDP-fucose and/or LNT, which is a reaction substrate for α1,2-fucosyltransferase. Therefore, in one embodiment of the present invention, the parent strain is preferably a genetically modified microorganism comprising at least one nucleotide sequence selected from a nucleotide sequence encoding rcsA (Accession Number: BAA15776.1), a nucleotide sequence encoding mannose-6-phosphate isomerase (Accession Number: BAA15361.1), a nucleotide sequence encoding phosphomannomutase (Accession Number: BAA15901.1), a nucleotide sequence encoding mannose-1-phosphate guanylyltransferase (Accession Number: BAA15905.1), a nucleotide sequence encoding GDP mannose-4,6-dehydratase (Accession Number: BAA15909.1), a nucleotide sequence encoding GDP-L-fucose synthase (Accession Number: BAA15908.1), a nucleotide sequence encoding lacY (Accession Number: BAE76125.1), a nucleotide sequence encoding galT (SEQ ID NO: 29), a nucleotide sequence encoding lgtA (SEQ ID NO: 31), a nucleotide sequence encoding glmS (Accession Number: BAE77559.1), a nucleotide sequence encoding glmM (Accession Number: BAE77220.1), a nucleotide sequence encoding glmU (Accession Number: BAE77558.1), a nucleotide sequence encoding Pgm (Accession Number: BAA35337.1), a nucleotide sequence encoding galU (Accession Number BAA36104.1), a nucleotide sequence encoding galE (Accession Number: BAA35421.1), a nucleotide sequence encoding galF (Accession Number: BAA15896.1), and a nucleotide sequence encoding pgi (Accession Number: BAE78027.1).

Particularly, the parent strain is more preferably a genetically modified microorganism comprising a nucleotide sequence encoding lacY, a nucleotide sequence encoding rcsA, a nucleotide sequence encoding galT, and a nucleotide sequence encoding lgtA. In one embodiment of the present invention, the genetically modified microorganism preferably has an enhanced ability to produce GDP-fucose and/or LNT as compared with a parent strain that is not genetically modified.

A known method may be used as a method for producing a microorganism having at least one activity selected from a lacY activity, a rcsA activity, a galT activity, a lgtA activity, a glmS activity, a glmM activity, a glmU activity, a pgm activity, a galU activity, a galE activity, a galF activity, and a pgi activity, or having an enhanced activity thereof. Specific examples thereof include methods using various genetic manipulations (Syst Microbiol Biomanufact, 2021, 1, 291).

Further, as described above, the parent strain preferably has a reduced or deleted lacZ activity and/or cholanic acid synthesis activity.

Accordingly, in one embodiment of the present invention, the parent strain is preferably a genetically modified microorganism having a reduced or deleted lacZ activity and/or cholanic acid synthesis activity, and more preferably a genetically modified microorganism not comprising the nucleotide sequence encoding lacZ and/or the nucleotide sequence encoding a wcaJ, wzxC, wcaK, wcaL, or wcaM gene which is a nucleotide sequence encoding a protein associated with cholanic acid synthesis.

In one embodiment of the present invention, the genetically modified microorganism preferably has an enhanced ability to produce GDP-fucose and/or LNT as compared with a parent strain that is not genetically modified.

A known method may be used as a method for producing Escherichia coli having a reduced or deleted β-galactosidase activity and/or cholanic acid synthesis activity. Specific examples include methods using various genetic manipulations (Metabolic Engineering, 2017, 41: 23-38).

Examples of the microorganism having an enhanced activity of the protein according to any one of the above [1] to [3] as compared with the microorganism of the parent strain include a microorganism having an increased copy number of the gene as compared with the parent strain, which is obtained by transforming the microorganism of the parent strain with a recombinant DNA containing a DNA encoding the protein.

Examples of the microorganism having an increased copy number of the gene as compared with the parent strain, which is obtained by transforming the microorganism of the parent strain with a recombinant DNA containing a DNA encoding the protein according to any one of the above [1] to [3] include a microorganism having an increased copy number of the gene on a chromosomal DNA, which is obtained by transforming the microorganism of the parent strain with the recombinant DNA containing the DNA encoding the protein according to any one of the above [1] to [3], and a microorganism carrying the above gene outside of a chromosomal DNA as a plasmid DNA.

The DNA encoding the protein according to any one of the above [1] to [3] may be any DNA as long as it encodes a protein having an activity of the protein according to any one of [1] to [3]. Specific examples thereof include one DNA selected from the group consisting of the following [4] to [7].
[4] A DNA encoding the protein according to any one of the above [1] to [3]
[5] A DNA consisting of the nucleotide sequence represented by SEQ ID NO: 1, 3, 5, 7, 9, 13, 17, or 25
[6] A DNA hybridizing with a DNA consisting of a nucleotide sequence complementary to the nucleotide sequence represented by SEQ ID NO: 1, 3, 5, 7, 9, 13, 17, or 25 under stringent conditions and encoding a homologous protein having an α1,2-fucosyltransferase activity
[7] A DNA consisting of a nucleotide sequence having an identity of 95% or more, preferably 97% or more, more preferably 98% or more, and most preferably 99% or more with the nucleotide sequence represented by SEQ ID NO: 1, 3, 5, 7, 9, 13, 17, or 25 and encoding a homologous protein having an α1,2-fucosyltransferase activity

In the above [6], the term "hybridizing" means that a DNA hybridizes to a DNA having a specific nucleotide sequence or a part of the DNA. Accordingly, the DNA having a specific nucleotide sequence or a part thereof is a DNA that can be used as a probe for Northern or Southern blot analysis, or a DNA that can be used as an oligonucleotide primer for PCR analysis.

Examples of the DNA used as a probe include a DNA having at least 100 bases or more, preferably 200 bases or more, and more preferably 500 bases or more. Examples of the DNA used as a primer include a DNA having at least 10 bases or more, and preferably 15 bases or more.

A method for a DNA hybridization experiment is well known, and for example, those skilled in the art can determine hybridization conditions according to the present description. The hybridization conditions can be determined according to those described in Molecular Cloning, 4th Edition (2012), Methods for General and Molecular Bacteriology, ASM Press (1994), and Immunology methods manual, Academic press(1996) as well as many other standard textbooks.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mmol/L sodium chloride, 75 mmol/L sodium citrate), 50 mmol/L sodium phosphate (pH 7.6), a 5 × Denhardt's solution, 10% dextran sulfate, and a 20 µg/l of denatured salmon sperm DNA, and then the filter is washed in a 0.2×SSC solution at, for example, about 65°C.

Examples of the DNA capable of hybridizing under the above stringent conditions include a DNA having an identity of at least 95% or more, preferably 97% or more, more preferably 98% or more, and most preferably 99% or more with the DNA consisting of the nucleotide sequence represented by SEQ ID NO: 1, 3, 5, 7, 9, 13, 17, or 25 when calculated based on the parameters or the like using BLAST, FASTA, or the like.

The DNA encoding the protein of the above [1] can be obtained by, for example, Southern hybridization for a chromosomal DNA library of a microorganism, preferably a microorganism using a probe DNA that can be designed based on the nucleotide sequence represented by SEQ ID NO: 1, 3, 5, 7, 9, 13, 17, or 25, or PCR [PCR protocols, Academic Press (1990)] using, as a template, a chromosomal DNA of the microorganism and using a primer DNA that can be designed based on the nucleotide sequence. The origin of the chromosomal DNA of the microorganism used in the above-described procedure is not particularly limited, and examples thereof include bacteria of the genus Neisseria (Neisseriaceae, Neisseriales), the genus Francisella, the genus Methylobacter, the genus Amphritea, the genus Sterolibacteriaceae, or the genus Helicobacter. Among them, the Francisella sp. FSC 1006 strain, the Neisseriaceae bacterium DSM 100970 strain, the Methylobacter tundripaludum strain, the Amphritea japonica strain, the Sterolibacteriaceae bacterium J5B strain, the Neisseriales bacterium strain, or the Helicobacter mustelae ATCC 43772 strain is preferred.

These strains can be available from public institutions, and the like. For example, the Francisella sp. FSC 1006 strain can be available from the Swedish Defence Research Agency. The Neisseriaceae bacterium DSM 100970 strain can be available from the University of Malaya. Further, the Methylobacter tundripaludum strain, the Amphritea japonica strain, and the Helicobacter mustelae ATCC 43772 strain can be available from the American Type Culture Collection (ATCC).

The DNA encoding the mutant protein of the above [2] can be obtained by, for example, performing error-prone PCR, or the like, using, as a template, a DNA consisting of the nucleotide sequence represented by SEQ ID NO: 1, 3, 5, 7, 9, 13, 17, or 25.

The DNA encoding the mutant protein in the above [2] can be obtained by PCR using a set of PCR primers each having, at the 5' end thereof, a nucleotide sequence designed to insert a target mutation (deletion, substitution, insertion, or addition) [Gene, 77, 51 (1989)].

The DNA can also be obtained by following an explanatory manual attached to a commercially available partially directed mutagenesis kit. Examples of the commercially available partially directed mutagenesis kit include a Prime STAR (registered trademark) Mutagenesis Basal Kit (manufactured by Takara Bio Inc.) capable of introducing a mutation (deletion, substitution, insertion, or addition) at a position to which a desired mutation is to be introduced.

That is, first, a pair of mutagenesis primers having a 15-base overlap on the 5' side is designed using a plasmid comprising a nucleotide sequence designed to introduce a desired mutation (deletion, substitution, insertion, or addition) as a template. At this time, the overlap portion includes a desired mutation. Next, PCR is performed using the mutagenesis primers and using a plasmid comprising a nucleotide sequence into which a desired mutation is introduced as a template. When the amplified fragment thus obtained is transformed into Escherichia coli, a plasmid comprising a nucleotide sequence into which a desired mutation is introduced is obtained.

The DNA encoding the homologous protein of the above [3] and the DNA of the above [6] and [7] can be obtained, for example, by a method same as the above method for obtaining the DNA by, for example, searching various gene sequence databases for a nucleotide sequence having an identity of 95% or more, preferably 97% or more, further preferably 98% or more, and most preferably 99% or more with the nucleotide sequence represented by SEQ ID NO: 1, 3, 5, 7, 9, 13, 17, or 25 searching various protein sequence databases for an amino acid sequence having an identity of 95% or more, preferably 97% or more, further preferably 98% or more, and most preferably 99% or more with the amino acid sequence represented by SEQ ID NO: 2, 4, 6, 8, 10, 14, 18, or 26, and using a probe DNA or a primer DNA that can be designed based on the nucleotide sequence or the amino acid sequence obtained by the search, and a microorganism having the DNA.

A nucleotide sequence of the DNA can be determined by using the obtained DNA according to any one of [4] to [7] as it is or cleaving the DNA with an appropriate restriction enzyme or the like, incorporating the DNA into a vector by an ordinary method, introducing the obtained recombinant DNA into a host cell, and then analyzing the DNA using a nucleotide sequence analysis method generally used, such as a dideoxy method [Proc. Natl. Acad. Sci., USA, 74, 5463 (1977)] or a nucleotide sequence analyzer such as an Applied Biosystems 3500 Genetic Analyzer and an Applied Biosystems 3730 DNA Analyzer (both manufactured by Thermo Fisher Scientific K.K.).

The host cell that can be used for determining the nucleotide sequence of the DNA may be any cell as long as the vector can be introduced and proliferated, and examples thereof include Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli XI,1-Blue and Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

Examples of the above vector include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT (Nucleic Acids Res., 18, 6069, 1990).

As a method for introducing the recombinant DNA, any method for introducing a DNA into a host cell can be used, and examples thereof include a method using calcium ions [Proc. Natl. Acad. Sci., USA, 69, 2110 (1972)], a protoplast method (JPS63-248394A), and an electroporation method [Nucleic Acids Res., 16, 6127 (1988)].

When the obtained DNA is partial length as a result of determining the nucleotide sequence, the full-length DNA can be obtained by a Southern hybridization method or the like for a chromosomal DNA library using the partial-length DNA as a probe.

Further, a target DNA can also be prepared by chemical synthesis using an NTS M series DNA synthesizer or the like manufactured by Nihon Techno Service Co., Ltd. based on the determined DNA nucleotide sequence.

The recombinant DNA containing the DNA encoding the protein according to any one of the above [1] to [3] refers to a recombinant DNA obtained by incorporating the DNA into an expression vector which is autonomously replicable in a parent strain or can be incorporated into a chromosome and contains a promoter at a position where the DNA can be transferred.

When the recombinant DNA is a recombinant DNA capable of being incorporated into a chromosome, the recombinant DNA may not contain a promoter.

The microorganism having an increased copy number of the gene as compared with the parent strain, which is obtained by transforming the microorganism of the parent strain with the recombinant DNA containing the DNA encoding the protein according to any one of the above [1] to [3] can be obtained by the following method.

Based on the DNA encoding the protein according to any one of the above [1] to [3] and obtained by the above method, a DNA fragment of an appropriate length containing a portion encoding the protein is prepared as necessary. A transformant having an improved production rate can be obtained by substituting a base such that a nucleotide sequence of the portion encoding the protein becomes an optimal codon for expression in a host cell.

A recombinant DNA is prepared by inserting the DNA fragment downstream of a promoter of an appropriate expression vector. By transforming the parent strain with the recombinant DNA, a microorganism having an increased copy number of a gene encoding the protein as compared with the parent strain can be obtained.

When a prokaryote such as bacteria is used as parent strain, the recombinant DNA is preferably a recombinant DNA composed of a promoter, a ribosomal binding sequence, the DNA according to any one of the above [4] to [7], and a transcription termination sequence. A gene for regulating the promoter may be contained.

It is preferable to use a plasmid in which a distance between a Shine-Dalgarno sequence, which is a ribosome binding sequence, and an initiation codon is adjusted to an appropriate distance (for example, 6 to 18 bases). In the recombinant DNA, a transcription termination sequence is not necessarily required for expression of the DNA, but it is preferable to place the transcription termination sequence immediately after a structural gene.

An expression level of the protein having an α1,2-fucosyltransferase activity can be improved by substituting a base such that the nucleotide sequence of the portion encoding the protein having an α1,2-fucosyltransferase activity becomes an optimal codon for host expression. Examples of the protein having an α1,2-fucosyltransferase activity include the protein according to any one of the above [1] to [3]. Information on the frequency of codon use in the parent strain used in the present invention can be obtained through a public database.

The expression vector is not particularly limited as long as it is an appropriate nucleic acid molecule for introducing the target DNA into a host and causing the target DNA to be amplified and expressed. For example, not only plasmids, but also, for example, artificial chromosomes, vectors using transposons, and cosmids may be used.

When a microorganism belonging to the genus Escherichia is used as the parent strain, examples of the expression vector include pColdI, pSTV28, pSTV29, pUC118 (all manufactured by Takara Bio Inc.), pMW118 and pMW119 (all manufactured by Nippon Gene Co., ltd.), pET21a, pCOLADuet-1, pCDFDuet-1, pCDF-1b, pRSF-1b (all manufactured by Merck Millipore Inc.), pMAL-c5x (manufactured by New England Biolabs), pGEX-4T-1, pTrc99A (both manufactured by GE Healthcare Bioscience), pTrcHis, pSE280 (both manufactured by Thermo Fisher Scientific K.K.), pGEMEX-1 (manufactured by Promega Corporation), pQE-30, pQE80L (both manufactured by Qiagen), pET-3, pBluescriptII SK(+), pBluescriptII KS(-) (all manufactured by Agilent Technologies, Inc.), pUAKQE31 (Appl. Environ. Microbiol. 2007, 73: 6378-6385), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK(+), pBluescript II KS(-) (manufactured by Stratagene Corporation), pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol.73, No.20, p 6378-6385], pPAC31 (WO1998/12343), pUC19 [Gene, 33, 103 (1985)], pPA1 (JPS63-233798A), and pKD46 [Proc. Natl. Acad. Sci., USA, 97, 6640-6645 (2000)].

The promoter in the case of using the above expression vector may be any promoter as long as it functions in cells of microorganisms belonging to the genus Escherichia, and for example, a promoter of a gene associated with amino acid biosynthesis, such as a trp promoter or an ilv promoter, and a promoter derived from, for example, an Escherichia coli or a phage, such as a uspApromoter, a lac promoter, a PL promoter, a PR promoter, or a PSE promoter can be used. Examples thereof include a promoter which is artificially modified in design, such as a promoter with two trp promoters in series, a tac promoter, a trc promoter, a lacT7 promoter, or a letIpromoter.

When a microorganism belonging to the genus Corynebacterium is used as the parent strain, examples of the expression vector include pCG1 (JPS57-134500A), pCG2 (JPS58-35197A), pCG4 (JPS57-183799A), pCG11 (JPS57-134500A), pCG116, pCE54, and pCB101 (all JPS58-105999A), pCE51, pCE52, and pCE53 [all Molecular and General Genetics, 196, 175, (1984)].

When the above expression vector is used, the promoter may be any promoter as long as it functions in cells of microorganisms belonging to the genus Corynebacterium, and for example, a P54-6 promoter [Appl. Microbiol. Biotechnol., 53, 674-679 (2000)] can be used.

When a yeast strain is used as the parent strain, examples of the expression vector include YEp13 (ATCC 37115), YEp24 (ATCC 37051), YCp50 (ATCC 37419), pHS19, and pHS15.

The promoter in the case of using the above expression vector may be any promoter as long as it functions in cells of the yeast strain, and examples thereof include a PHOS promoter, a PGK promoter, a GAP promoter, an ADH promoter, a gall promoter, a gal10 promoter, a heat shock polypeptide promoter, an MFα1 promoter, and a CUP1 promoter.

By inserting the DNA fragment according to any one of the above [4] to [7] downstream of a promoter of an appropriate expression vector, a recombinant DNA to be used in the production method of the present invention can be prepared.

Examples of a method for introducing a recombinant DNA into a parent strain as an autonomously replicable plasmid include a method using calcium ions [Proc. Natl. Acad. Sci., USA, 69, 2110 (1972)], a protoplast method (JPS63-248394A), and an electroporation method [Nucleic Acids Res., 16, 6127 (1988)].

Examples of the method for incorporating a recombinant DNA into a chromosome of a host cell include a homologous recombination method. Examples of the homologous recombination method include a method using a plasmid for homologous recombination that can be prepared by linking with a plasmid DNA having a drug resistance gene that cannot autonomously replicate in a host cell to be introduced. Examples of the method using homologous recombination frequently used in Escherichia coli include a method of introducing a recombinant DNA using a homologous recombination system of a lambda phage [Proc. Natl. Acad. Sci. USA, 97, 6640-6645 (2000)].

Further, by using a selection method based on the fact that Escherichia coli becomes sucrose-sensitive due to a Bacillus subtilis revansucrase incorporated on the chromosome together with a recombinant DNA, or a selection method based on the fact that Escherichia coli becomes streptomycin-sensitive by incorporating a wild-type rpsL gene into Escherichia coli comprising a mutant rpsL gene for streptomycin resistance [Mol. Microbiol., 55, 137 (2005), Biosci. Biotechnol. Biochem., 71, 2905 (2007)], Escherichia coli with a target region on a chromosomal DNA of the host cell substituted with the recombinant DNA can be obtained.

The fact that the recombinant DNA is introduced into the parent strain as an autonomously replicable plasmid or incorporated into a chromosome of the parent strain can be confirmed by, for example, a method in which a gene originally contained in a chromosomal DNA of a microorganism cannot be amplified, but the gene introduced through transformation can be amplified by PCR using a primer set to confirm an amplification product. The fact that the transcription amount of the DNA or the production amount of the protein encoded by the DNA is increased can be confirmed by a method of comparing the transcription amount of the gene in the microorganism with that of the parent strain by Northern blotting, or the production amount of the protein in the microorganism with that of the parent strain by Western blotting.

The fact that the microorganism produced by the above method is a microorganism having an enhanced activity of the protein according to any one of the above [1] to [3] and having improved productivity of LNFPI as compared with a parent strain can be confirmed by appropriately diluting a culture solution after culturing the microorganism, centrifuging the culture solution, and analyzing LNFPI contained in a supernatant or inside bacterial cells using a carbohydrate analyzer or a high performance liquid chromatograph mass spectrometer to be described later, thereby comparing with that of the parent strain.

The above-described microorganism has an enhanced activity of the protein according to any one of the above [1] to [3] as compared with a parent strain, and therefore can selectively transfer fucose to the N-acetylglucosamine site of LNT, thereby improving the productivity of LNFPI. Examples of such a microorganism include an NNN/pGsFucT strain having enhanced expression of a GsFucT gene, an NNN/pFsFucT strain having enhanced expression of a FsFucT gene, an NNN/pNbFucT1 strain having enhanced expression of a NbFucT1 gene, an NNN/pMtFucT strain having enhanced expression of an MtFucT gene, an NNN/pAjFucT strain having enhanced expression of a AjFucT gene, an NNN/pSbFucT strain having enhanced expression of an SbFucT gene, an NNN/pPsFucT strain having enhanced expression of a PsFucT gene, an NNN/pNbFucT2 strain having enhanced expression of a NbFucT2 gene, and an NNN/pHMFT strain having enhanced expression of an HMFT gene, which will be described later in Examples.

In a microorganism having enhanced expression of GsFucT, FsFucT, NbFucT1, MtFucT, AjFucT, SbFucT, NbFucT2, or HMFT, which is an example of such a microorganism, the α1,2-fucose transferase activity capable of selectively transferring fucose to an N-acetylglucosamine site is enhanced, and the productivity of LNFPI can be improved. Therefore, LNFPI can be efficiently produced by using these microorganisms. These microorganisms can also be used to produce fucosylated oligosaccharides other than LNFPI, such as fucosyllactose such as 2'FL and 3'FL.

### <Method for Producing Fucose-containing Carbohydrate>

Examples of a method for producing a fucose-containing carbohydrate of the present invention (hereinafter, also abbreviated as method of the present invention) include a method for producing a fucose-containing carbohydrate including: preparing the above transformant and producing oligosaccharides in a culture using the transformant. In the method of the present invention, the fucose-containing carbohydrate is preferably LNFPI.

The above method for culturing the transformant can be performed according to a method generally used for culturing a microorganism. As a culture medium for culturing the transformant, any of a natural culture medium and a synthetic culture medium may be used as long as the culture medium contains a carbon source, a nitrogen source, an inorganic salt, and the like that can be assimilated by the microorganism and can efficiently culture the transformant.

Any carbon source may be used as long as it can be assimilated by the microorganism, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolyzate, a soybean meal, a soybean meal hydrolyzate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate.

As the transformant used in the method for producing a fucose-containing carbohydrate, a microorganism having an ability to produce glucose, lactose, lactose monohydrate, or the like may be used.

In the method for producing a fucose-containing carbohydrate, glucose, lactose, lactose monohydrate, and the like may be added to the culture medium during culture.

When the transformant used in the method for producing a fucose-containing carbohydrate does not have the ability to produce GDP-fucose and/or LNT, GDP-fucose or LNT may be added to the culture medium.

In the method for producing a fucose-containing carbohydrate, instead of adding glucose, lactose, lactose monohydrate, or LNT to the culture medium during the culture, glucose, lactose, lactose monohydrate, or LNT may be supplied to the transformant of the present invention by culturing a microorganism having an ability to produce glucose, lactose, lactose monohydrate, or LNT from a saccharide simultaneously with the transformant of the present invention.

In the method for producing a fucose-containing carbohydrate, β-galactosidase and WcaJ are preferably absent in the culture medium.

Culture is generally preferably performed under aerobic conditions such as shaking culture, submerged aeration agitation culture, or deep aeration stirring culture. The culture temperature is generally 30°C to 37°C, and the culture time is generally 24 hours to 3 days. The pH of the culture solution during culture is generally maintained at 6.0 to 8.0. The pH is adjusted using an inorganic or organic acid, an alkaline solution, urea, calcium carbonate, ammonia, or the like.

A fucose-containing carbohydrate can be produced by producing the fucose-containing carbohydrate in a culture by the above culture.

Generally, the fucose-containing carbohydrate can be collected from a supernatant after centrifugation of the culture. When the fucose-containing carbohydrate is accumulated inside bacterial cells, for example, the fucose-containing carbohydrate can be collected, by an ion exchange resin method, from a supernatant obtained by crushing the bacterial cells by ultrasonic waves or the like and removing the bacterial cells by centrifugation.

A desired fucose-containing carbohydrate can be produced by adding other saccharides to the fucose-containing carbohydrate in the culture or the collected fucose-containing carbohydrate.

### [Analysis Example]

### (1) Analysis and Quantification of LNFPI, 2'FL, or Lactose

In Examples, analysis and quantification of LNFPI, 2'FL, or lactose were performed according to the procedures shown below.

A culture solution containing microorganisms after culture was centrifuged, and a supernatant was collected. Precipitated bacterial cells were suspended in water in an amount equal to that of the original culture solution, further, the bacterial cells were disrupted by adding an equal amount of chloroform, followed by centrifugation, and the obtained supernatant aqueous phase was used as an intracellular fraction. LNFPI, 2'FL, and lactose contained in the supernatant or the intracellular fraction were analyzed by a carbohydrate analyzer ICS-5000 (manufactured by Thermo Fisher Scientific K.K.).

### [Analysis Conditions]

Column: CarboPAC PA1
Column temperature: 25°C

### Mobile phase:

(mobile phase A) water
(mobile phase B) 500 mmol/L sodium hydroxide
(mobile phase C) 300 mmol/L sodium acetate

### Mixing ratio of mobile phase A, mobile phase B, and mobile phase C:

(0 minutes to 10 minutes) 80:20:0
(10 minutes to 18 minutes) gradient from 80:20:0 to 70:20:10
(18 minutes to 35 minutes) gradient from 70:20:10 to 0:20:80
(35 minutes to 40 minutes) 0:20:80
(40 minutes to 50 minutes) 80:20:0
Flow rate: 1.0 mL/min
Detector: pulsed amperometric detector

### (2) Analysis and Quantification of LNFPI, LNTII, or LNT

In Examples, analysis and quantification of LNFPI, LNTII, or LNT were performed by the following procedure.

A supernatant and an intracellular fraction were prepared from a culture solution containing cultured microorganisms in the same manner as in the above (1). LNFPI, LNTII, and LNT contained in the supernatant or the intracellular fraction were analyzed by UFLC & LCMS-8040 (manufactured by SHIMADZU).

### [Analysis Conditions]

Column: Co re gel 87H3 (7.8 × 300 mm)
Column temperature: 40°C
Mobile phase: 0.1% formic acid in water, isocratic elution
Measurement time: 25 minutes
Flow rate: 0.4 ml/min
Injection amount: 10 µL
Detection: SIM mode

### [Example]

Examples of the present invention are shown below, but the present invention is not limited to these Examples.

### [Example 1] Construction of Microorganisms Expressing Various Types of α1,2-Fucosyltransferase

### (1) Construction of Host Strain

### <Acquisition of DNA Fragment to be Used as Marker for Gene Deletion>

PCR was performed using, as a primer set, a DNA consisting of the nucleotide sequences represented by SEQ ID NOs: 37 and 38 and using, as a template, pCatSac (Appl Environ Microbiol (2013) 79, 3033 -3039) to obtain a cat-sacB fragment containing a chloramphenicol-resistant cat gene and a sucrose sensitive sacB gene.

### <Construction of Escherichia Coli Lacking β-galactosidase Activity, Lactose Permease Activity, and Cholanic Acid Synthesis Activity>

Escherichia coli deficient in DNA encoding β-galactosidase (hereinafter, referred to as lacZ gene), DNA encoding lactose permease (hereinafter, referred to as lacY gene), and DNA encoding a cholanic acid production-related protein (hereinafter, referred to as wcaJ, wzxC, wcaK, wcaL, or wcaM gene) was constructed by the following method. Note that the lacZ and lacY (hereinafter, referred to as lacZY), and wcaJ, wzxC, wcaK, wcaL, and wcaM (hereinafter, referred to as wcaJ-wzxC-wcaKLM) each form an operon on the Escherichia coli genome.

PCR was performed using, as a template, a genomic DNA of an Escherichia coli W3110 strain prepared by an ordinary method and using, as a primer set, a DNA consisting of the nucleotide sequences represented by "Primer set" in Table 1 to amplify each DNA fragment.

**[Table 1]**

| Primer set (SEQ ID NOs:) | Amplified DNA fragment | Remarks |
|---|---|---|
| 39 and 40 | lacZ upstream 1 | Sequences in nucleotide sequences represented by SEQ ID NOs: 37 and 39 at 5' ends are complementary |
| 41 and 42 | lacY downstream 1 | Sequences in nucleotide sequences represented by SEQ ID NOs: 38 and 41 at 5' ends are complementary |
| 40 and 43 | lacZ upstream 2 | Sequences in nucleotide sequences represented by SEQ ID NOs: 43 and 44 at 5' ends are complementary |
| 42 and 44 | lacY downstream 2 | |

The lacZ upstream 1 and lacZ upstream 2 include a region from an initiation codon of the lacZ gene to about 1000 bp upstream of the initiation codon. The lacY downstream 1 and lacY downstream 2 contain a region from about 50 bp to about 1000 bp downstream of a stop codon of the lacY gene.

PCR was performed using, as a template, a mixture of the lacZ upstream 1, the lacY downstream 1, and the cat-sacB fragment at an equimolar ratio and using, as a primer set, a DNA consisting of the nucleotide sequences represented by SEQ ID NOs: 40 and 42 to obtain a DNA (hereinafter, referred to as lacZY::cat-sacB) fragment consisting of a sequence with the cat-sacB fragment inserted in a region around the lacZ and lacY genes.

PCR was performed using, as a template, a mixture of the lacZ upstream 2 and the lacY downstream 2 at an equimolar ratio and using, as a primer set, a DNA consisting of the nucleotide sequences represented by SEQ ID NOs: 40 and 42 to obtain a DNA (hereinafter, referred to as ΔlacZY) fragment consisting of a sequence with the lacZ upstream and the lacY downstream directly linked to each other without lacZY

The lacZY::cat-sacB fragment was introduced into a W3110S3GK strain (NBRC114657) carrying a plasmid pKD46 containing a gene encoding λ recombinase [Datsenko, K. A., Warner, B. L. L., Proc. Natl. Acad. Sci, USA, Vol. 97, 6640-6645, (2000)] by an electroporation method to obtain a transformant (a transformant with the lacZY gene substituted with lacZY::cat-sacB) exhibiting chloramphenicol resistance and a sucrose sensitivity.

The ΔlacZY fragment was introduced into the transformant by the electroporation method to obtain a transformant (a transformant with lacZY::cat-sacB substituted with ΔlacZY) exhibiting chloramphenicol sensitivity and sucrose resistance. Among them, a transformant (a transformant with pKD46 lost) exhibiting an ampicillin sensitivity was further obtained. The transformant was named W3110S3GKΔIacZY

Similarly, PCR was performed using, as a template, the genomic DNA of the W3110 strain and using, as a primer set, a DNA consisting of the nucleotide sequences represented by "Primer set" in Table 2 to obtain each amplified DNA fragment.

**[Table 2]**

| Primer set (SEQ ID NOs:) | Amplified DNA fragment | Remarks |
|---|---|---|
| 45 and 46 | wcaJ upstream 1 | Sequences in nucleotide sequences represented by SEQ ID NOs: 37 and 45 at 5' ends are complementary |
| 47 and 48 | wcaM downstream 1 | Sequences in nucleotide sequences represented by SEQ ID NOs: 38 and 47 at 5' ends are complementary |
| 46 and 49 | wcaJ upstream 2 | Sequences in nucleotide sequences represented by SEQ ID NOs: 49 and 50 at 5' ends are complementary |
| 48 and 50 | wcaM downstream 2 | |

The wcaJ upstream 1 and the wcaJ upstream 2 contain a region from an initiation codon of the wcaJ gene to about 1000 bp upstream of the initiation codon. The wcaM downstream 1 and the wcaM downstream 2 contain a region from a stop codon of the wcaM gene to about 1000 bp downstream of the stop codon.

PCR was performed using, as a template, a mixture of the wcaJ upstream 1, the wcaM downstream 1, and the cat-sacB fragment at an equimolar ratio and using, as a primer set, a DNA consisting of the nucleotide sequences represented by SEQ ID NOs: 46 and 48 to obtain a DNA (hereinafter, referred to as wcaJ-wzxC-wcaKLM::cat-sacB) fragment consisting of a sequence with the cat-sacB fragment inserted in a region around a wcaJ-wzxC-wcaKLM operon.

PCR was performed using, as a template, a mixture of the wcaJ upstream 2 and the wcaM downstream 2 at an equimolar ratio and using, as a primer set, a DNA consisting of the nucleotide sequences represented by SEQ ID NOs: 46 and 48 to obtain a DNA (hereinafter, referred to as ΔwcaJ-wzxC-wcaKLM) fragment consisting of a sequence with the wcaJ upstream and the wcaM downstream directly linked to each other without wcaJ-wzxC-wcaKLM.

The wcaJ-wzxC-wcaKLM::cat-sacB fragment was introduced into the W3110S3GKΔlacZY constructed as described above by the electroporation method to obtain a transformant (a transformant with wcaJ-wzxC-wcaKLM substituted with wcaJ-wzxC-wcaKLM::cat-sacB) exhibiting chloramphenicol resistant and sucrose sensitivity.

The ΔwcaJ-wzxC-wcaKLM fragment was introduced into the transformant by the electroporation method to obtain a transformant (a transformant with wcaJ-wzxC-wcaKLM::cat-sacB substituted with ΔwcaJ-wzxC-wcaKLM) exhibiting chloramphenicol sensitivity and sucrose resistance. Further, a transformant (a transformant without pKD46) exhibiting ampicillin sensitivity was obtained. The transformant was named W3110 S3 GKΔlacZYΔwcaJM.

### <Construction of Microorganism having Enhanced Expression of β1,3-Galactosyltransferase and β1,3-N-acetylglucosaminetransferase>

Escherichia coli having a gene encoding β1,3-galactosyltransferase (hereinafter, referred to as Cvβ3GalT) derived from the Chromobacterium violaceum ATCC 553 strain and consisting of the amino acid sequence represented by SEQ ID NO:34, a gene encoding β1,3-N-acetylglucosaminetransferase (hereinafter, referred to as NpLgtA) derived from the Neisseria polysaccharea ATCC 43768 and represented by SEQ ID NO:36, and the lacY gene derived from the W3110 strain arranged under the uspA promoter, and having a plasmid for expressing the genes was constructed by the following method.

PCR was performed using, as a primer set, a DNA consisting of the nucleotide sequences represented by "Primer set" in Table 3 and using, as a template, a DNA described in "Template" in Table 3 to obtain each amplified DNA fragment.

**[Table 3]**

| Primer Set (SEQ ID NOs:) | Template | Amplified DNA fragment |
|---|---|---|
| 51 and 52 | DNA represented by SEQ ID NO: 33 | Cvβ3galT (SEQ ID NO: 33) |
| 53 and 54 | DNA represented by SEQ ID NO: 35 | NplgtA (SEQ ID NO: 35) |
| 55 and 56 | Genomic DNA of Escherichia coli W3110 strain | lacY |

The DNA represented by SEQ ID NO: 33 was a DNA in which a nucleotide sequence of the gene encoding the β1,3-galactosyltransferase Cvβ3GalT derived from the Chromobacterium violaceum ATCC 553 strain described in ACS Catal. 2019, 9(12), 10721-10726 was codon-optimized for expression in Escherichia coli, and was prepared by artificial synthesis.

The DNA represented by SEQ ID NO: 35 was a DNA in which a nucleotide sequence of the gene encoding β1,3-N-acetylglucosaminetransferase NpLgtA derived from the Neisseria polysaccharea ATCC 43768 strain and represented by SEQ ID NO: 36 was codon-optimized for expression in Escherichia coli, and was prepared by artificial synthesis. The nucleotide sequences represented by SEQ ID NOs: 52 and 53 and SEQ ID NOs: 54 and 55 each comprise a complementary sequence at the 5' end.

PCR was performed using, as a template, a mixture of a Cvβ3galT fragment, a NplgtA fragment, and a lacY fragment at an equimolar ratio and using, as a primer set, a DNA consisting of the nucleotide sequences represented by SEQ ID NOs: 51 and 56 to obtain a DNA fragment with the three fragments linked (hereinafter, referred to as Cvβ3galT-NplgtA-lacY).

PCR was performed using, as a primer set, an oligonucleotide consisting of the nucleotide sequences represented by SEQ ID NOs: 57 and 58 and using, as a template, a plasmid pUAKQE31 (Appl. Environ. Microbiol. 2007, 73: 6378-6385) to obtain a vector fragment of about 4.7 kb. The nucleotide sequences represented by SEQ ID NOs: 51 and 57 and SEQ ID NOs: 56 and 58 each comprise a complementary sequence at the 5' end.

The Cvβ3galT-NplgtA-lacY fragment and vector fragment obtained above were ligated using an In-Fusion HD Cloning Kit (manufactured by Takara Bio Inc.) to obtain an expression plasmid pUAKQE-Cvβ3galT-NplgtA-lacY. The above-constructed W3110S3GKΔlacZYΔwcaJM strain was transformed with the above expression plasmid pUAKQE-Cvβ3galT-NplgtA-lacY to construct Escherichia Coli having pUAKQE-Cvβ3galT-NplgtA-lacY, which was named NNN strain.

### (2) Construction of Microorganism having α1,2-Fucosyltransferase Activity

Using the NNN strain constructed in the above (1), Escherichia coli having a gene encoding various types ofα1,2-fucosyltransferase, and rcsA derived from the W3110 strain arranged under the lac promoter and having a plasmid for expressing the gene was constructed by the following method.

### <Construction of Expression Vector>

PCR was performed using, as a primer set, a DNA consisting of the nucleotide sequences represented by SEQ ID NOs:59 and 60 and using, as a template, the W3110 strain prepared by an ordinary method to obtain a rcsA fragment. PCR was performed using a plasmid pSTV29 (manufactured by Takara Bio Inc.) as a template and a DNA consisting of the nucleotide sequences represented by SEQ ID NOs: 61 and 62 as a primer set to obtain a vector fragment of about 2.9 kb. In this case, the nucleotide sequences represented by SEQ ID NOs: 59 and 61 and SEQ ID NOs: 60 and 62 each comprise a complementary sequence at the 5' end.

The rcsA fragment and vector fragment obtained above were ligated using an In-Fusion HD Cloning Kit (manufactured by Takara Bio Inc.) to obtain an expression vector pSTV-rcsA.

### <Construction of Plasmid for Expressing α1,2-Fucosyltransferase>

PCR was performed using, as a primer set, a DNA consisting of the nucleotide sequences represented by "Primer set" in Table 4 and using, as a template, a DNA described in "Template" in Table 4 to obtain each amplified DNA fragment.

**[Table 4]**

| Primer set (SEQ ID NOs:) | Template | Amplified DNA fragment |
|---|---|---|
| 63 and 64 | DNA represented by SEQ ID NO: 1 | GsFucT (SEQ ID NO: 1) |
| 65 and 66 | DNA represented by SEQ ID NO: 3 | FsFucT (SEQ ID NO: 3) |
| 67 and 68 | DNA represented by SEQ ID NO: 5 | NbFucT1 (SEQ ID NO: 5) |
| 69 and 70 | DNA represented by SEQ ID NO: 7 | MtFucT (SEQ ID NO: 7) |
| 71 and 72 | DNA represented by SEQ ID NO: 9 | AjFucT (SEQ ID NO: 9) |
| 73 and 74 | DNA represented by SEQ ID NO: 11 | PaFucT (SEQ ID NO: 11) |
| 75 and 76 | DNA represented by SEQ ID NO: 13 | SbFucT (SEQ ID NO: 13) |
| 77 and 78 | DNA represented by SEQ ID NO: 15 | PsFucT (SEQ ID NO: 15) |
| 79 and 80 | DNA represented by SEQ ID NO: 17 | NbFucT2 (SEQ ID NO: 17) |
| 81 and 82 | DNA represented by SEQ ID NO: 19 | CMfFucT (SEQ ID NO: 19) |
| 83 and 84 | DNA represented by SEQ ID NO: 21 | WbwK (SEQ ID NO: 21) |
| 85 and 86 | DNA represented by SEQ ID NO: 23 | WbiQ (SEQ ID NO: 23) |
| 87 and 88 | DNA represented by SEQ ID NO: 25 | HMFT (SEQ ID NO: 25) |

The DNA represented by SEQ ID NO: 1 was a DNA in which a nucleotide sequence of a gene encoding the α1,2-fucosyltransferase GsFucT derived from the Gramella sp. MAR_2010_147 strain and represented by SEQ ID NO:2 was codon-optimized for expression in Escherichia coli, and was prepared by artificial synthesis.

The DNA represented by SEQ ID NO: 3 was a DNA in which a nucleotide sequence of the gene encoding the α1,2-fucosyltransferase FsFucT derived from the Francisella sp. FSC1006 strain and represented by SEQ ID NO: 4 was codon-optimized for expression in Escherichia coli, and was prepared by artificial synthesis.

The DNA represented by SEQ ID NO: 5 was a DNA in which a nucleotide sequence of the gene encoding the α1,2-fucosyltransferase NbFucT1 derived from the Neisseriaceae bacterium DSM 100970 strain and represented by SEQ ID NO: 6 was codon-optimized for expression in Escherichia coli, and was prepared by artificial synthesis.

The DNA represented by SEQ ID NO: 7 was a DNA in which a nucleotide sequence of the gene encoding the α1,2-fucosyltransferase MtFucT derived from the Methylobacter tundripaludum strain and represented by SEQ ID NO: 8 was codon-optimized for expression in Escherichia coli, and was prepared by artificial synthesis.

The DNA represented by SEQ ID NO: 9 was a DNA in which a nucleotide sequence of the gene encoding the α1,2-fucosyltransferase AjFucT derived from the Amphritea japonica strain and represented by SEQ ID NO: 10 was codon-optimized for expression in Escherichia coli, and was prepared by artificial synthesis.

The DNA represented by SEQ ID NO: 11 was a DNA in which a nucleotide sequence of the gene encoding the α1,2-fucosyltransferase PaFucT derived from the Pseudohalocynthiibacter aestuariivivens strain and represented by SEQ ID NO: 12 was codon-optimized for expression in Escherichia coli, and was prepared by artificial synthesis.

The DNA represented by SEQ ID NO: 13 was a DNA in which a nucleotide sequence of the gene encoding the α1,2-fucosyltransferase SbFucT derived from the Sterolibacteriaceae bacterium J5B strain and represented by SEQ ID NO: 14 was codon-optimized for expression in Escherichia coli, and was prepared by artificial synthesis.

The DNA represented by SEQ ID NO: 15 was a DNA in which a nucleotide sequence of the gene encoding the α1,2-fucosyltransferase PsFucT derived from the Pedobacter sp. CF074 strain and represented by SEQ ID NO: 16 was codon-optimized for expression in Escherichia coli, and was prepared by artificial synthesis.

The DNA represented by SEQ ID NO: 17 was a DNA in which a nucleotide sequence of the gene encoding the α1,2-fucosyltransferase NbFucT2 derived from the Neisseriales bacterium strain and represented by SEQ ID NO: 18 was codon-optimized for expression in Escherichia coli, and was prepared by artificial synthesis.

The DNA represented by SEQ ID NO: 19 was a DNA in which a nucleotide sequence of the gene encoding the α1,2-fucosyltransferase CMfFucT derived from the Candidatus Methylobacter favarea strain and represented by SEQ ID NO: 20 was codon-optimized for expression in Escherichia coli, and was prepared by artificial synthesis.

The DNA represented by SEQ ID NO: 21 was a DNA in which a nucleotide sequence of the gene encoding the α1,2-fucosyltransferase WbwK derived from the Escherichia coli O86 strain and represented by SEQ ID NO: 22 was codon-optimized for expression in Escherichia coli, and was prepared by artificial synthesis.

The DNA represented by SEQ ID NO: 23 was a DNA in which a nucleotide sequence of the gene encoding the α1,2-fucosyltransferase WbiQ derived from the Escherichia coli O127 strain and represented by SEQ ID NO: 24 was codon-optimized for expression in Escherichia coli, and was prepared by artificial synthesis.

The DNA represented by SEQ ID NO: 25 was a nucleotide sequence of the gene encoding the α1,2-fucosyltransferase HMFT derived from the Helicobacter mustelae ATCC 43772 strain and represented by SEQ ID NO: 26, and was prepared by artificial synthesis.

PCR was performed using, as a template, the expression vector pSTV29-rcsA constructed above and using, as a primer set, a DNA consisting of the nucleotide sequences represented by SEQ ID NOs: 61 and 95 to obtain a vector fragment of about 3.5 kb.

The nucleotide sequences represented by SEQ ID NOs: 63, 65, 67, 69, 71, 73, 75, 77, 79, 81, 83, 85, 87, and 61, and SEQ ID NOs: 64, 66, 68, 70, 72, 74, 76, 78, 80, 82, 84, 86, 88, and 95 each comprise a complementary sequence at the 5' end.

The obtained each amplified DNA fragment and the vector fragment were ligated using an In-Fusion HD Cloning Kit (manufactured by Takara Bio Inc.) to construct plasmids expressing various types of α1,2-fucosyltransferase, pGsFucT, pFsFucT, pNbFucT1, pMtFucT, pAjFucT, pPaFucT, pSbFucT, pPsFucT, pNbFucT2, pCMfFucT, pWbwK, pWbiQ, and pHMFT.

### <Construction of Escherichia Coli having Plasmid for Expressing α1,2-Fucosyltransferase>

The NNN strain constructed in the above (1) was transformed with the above-obtained plasmid for expressing α1,2-fucosyltransferase and pSTV29-rcsA as a vector control to construct Escherichia Coli strains having various plasmids, which were named NNN/pGsFucT strain, NNN/pFsFucT strain, NNN/pNbFucT1 strain, NNN/pMtFucT strain, NNN/pAjFucT strain, NNN/pPaFucT strain, NNN/pSbFucT strain, NNN/pPsFucT strain, NNN/pNbFucT2 strain, NNN/pCMfFucT strain, NNN/pWbwK strain, NNN/pWbiQ strain, NNN/pHMFT strain, and NNN/pCtrl strain, respectively.

### [Comparative Example] Construction of Microorganism Expressing Known α1,2-Fucosyltransferase

A microorganism expressing α1,2-fucosyltransferase known to be capable of producing LNFPI was constructed by the following method.

PCR was performed using, as a primer set, a DNA consisting of the nucleotide sequences represented by "Primer set" in Table 5 and using, as a template, a DNA described in "Template" in Table 5 to obtain each amplified DNA fragment.

**[Table 5]**

| Primer set (SEQ ID NOs:) | Template | Amplified DNA fragment |
|---|---|---|
| 89 and 90 | DNA represented by SEQ ID NO: 27 | FucT54 (SEQ ID NO: 27) |
| 91 and 92 | DNA represented by SEQ ID NO: 29 | Te2FT (SEQ ID NO: 29) |
| 93 and 94 | DNA represented by SEQ ID NO: 31 | FutC (SEQ ID NO: 31) |

The DNA represented by SEQ ID NO: 27 was a DNA in which a nucleotide sequence of a gene encoding α1,2-fucosyltransferase FucT54 derived from the Sideroxydans lithotrophicus ES-11 strain and represented by SEQ ID NO: 28 was codon-optimized for expression in Escherichia coli, and was prepared by artificial synthesis.

The DNA represented by SEQ ID NO: 29 was a DNA in which a nucleotide sequence of a gene encoding α1,2-fucosyltransferase Te2FT derived from the Thermosynechococcus elongatus BP-1 strain and represented by SEQ ID NO: 30 was codon-optimized for expression in Escherichia coli, and was prepared by artificial synthesis.

The DNA represented by SEQ ID NO: 31 was a nucleotide sequence of a gene encoding α1,2-fucosyltransferase FutC derived from the Helicobacter pylori 26695 strain and represented by SEQ ID NO: 32, and was prepared by artificial synthesis.

PCR was performed using, as a template, the expression vector pSTV-rcsA constructed in Example 1(2) and using, as a primer set, a DNA consisting of the nucleotide sequences represented by SEQ ID NOs: 61 and 95 to obtain a vector fragment of about 3.5 kb. The nucleotide sequences represented by SEQ ID NOs: 89, 91, 93, and 61, and SEQ ID NOs: 90, 92, 94, and 95 each contain a complementary sequence at the 5' end.

The above-obtained each amplified DNA fragment and the vector fragment were linked using an In-Fusion HD Cloning Kit (manufactured by Takara Bio Inc.) to construct plasmids expressing various types of α1,2-fucosyltransferase, pFucT54, pTe2FT, and pFutC.

The NNN strain constructed in Example 1(1) was transformed with the above-obtained plasmid for expressing α1,2-fucosyltransferase to construct Escherichia coli strains having various plasmids, which were named NNN/pFucT54 strain, NNN/pTe2FT strain, and NNN/pFutC strain, respectively.

### [Example 2] Evaluation of Productivity of LNFPI

Productivity of LNFPI was evaluated for the above NNN/pGsFucT strain, NNN/pFsFucT strain, NNN/pNbFucT1 strain, NNN/pMtFucT strain, NNN/pAjFucT strain, NNN/pPaFucT strain, NNN/pSbFucT strain, NNN/pPsFucT strain, NNN/pNbFucT2 strain, NNN/pCMfFucT strain, NNN/pWbwK strain, NNN/pWbiQ strain, and NNN/pHMFT strain obtained in Example 1(2). As a positive control, the NNN/pTe2FT strain and NNN/pFutC strain constructed in Comparative Example were used. As a negative control, the NNN/pCtrl strain constructed in Example 1(2) was used.

Each strain was cultured on an LB plate containing 100 mg/L kanamycin and 25 mg/L chloramphenicol at 37°C for 18 hours, then inoculated into a plastic test tube containing 2 mL of LB culture medium containing 100 mg/L kanamycin and 25 mg/L chloramphenicol, and cultured with shaking at 30°C for 15 hours. Thereafter, 0.2 mL of each obtained culture solution was inoculated into a large-sized test tube containing 4 mL of a production culture medium [glucose 30 g/L, lactose monohydrate 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (adjusted to pH 7.2 by aqueous sodium hydroxide, except for glucose, lactose monohydrate, and magnesium sulfate heptahydrate, and then autoclaved) (aqueous solutions containing glucose, lactose monohydrate, and magnesium sulfate heptahydrate were separately prepared, autoclaved, cooled, and then mixed)] containing 100 mg/L of kanamycin and 25 mg/L of chloramphenicol, and cultured with shaking at 30°C for 29 hours.

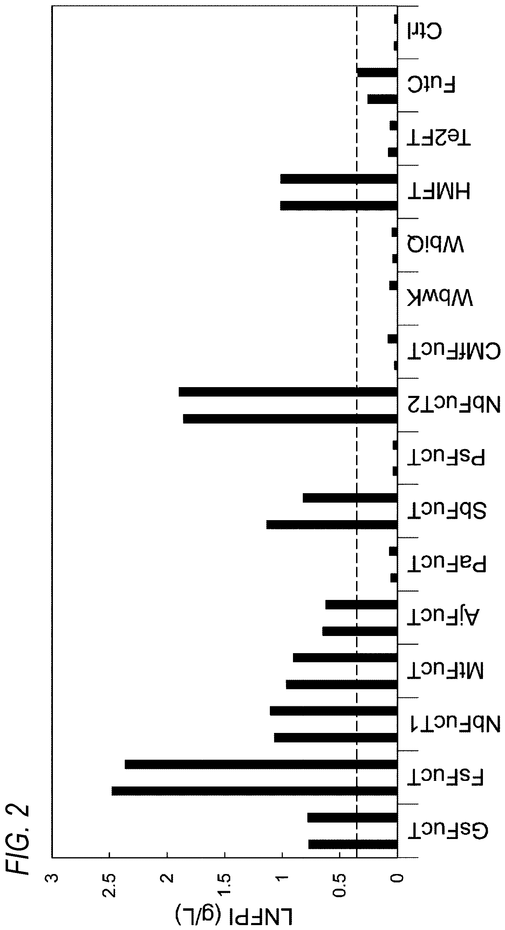

After completion of the culture, a culture solution was centrifuged and appropriately diluted, and LNFPI, LNTII, or LNT contained in the supernatant and the intracellular fraction was analyzed by UFLC & LCMS-8040. The results are shown in Table 6. FIG. 2 shows results of combined amounts of LNFPI produced in the supernatant and the intracellular fraction.

**[Table 6]**

| Strain name | LNFPI [g/L] | | LNTII [g/L] | | LNT [g/L] | |
|---|---|---|---|---|---|---|
| | Supernatant | Inside bacterial cells | Supernatant | Inside bacterial cells | Supernatant | Inside bacterial cells |
| NNN/pGsFucT | 0.19 | 0.58 | 1 0.26 | 0.03 | 0.76 | 1.47 |
| NNN/pFsFucT | 0.65 | 1.96 | 0.23 | 0.02 | 0.18 | 1 0.21 |
| NNN/pNbFucT1 | 0.16 | 0.93 | 0.09 | 0.01 | N.D | 0.03 |
| NNN/pMtFucT | 0.15 | 0.79 | 0.18 | 0.03 | 0.14 | 1 0.38 |
| NNN/pAjFucT | 0.11 | 0.52 | 0.12 | 0.01 | 0.21 | 0.54 |
| NNN/pPaFucT | 0.01 | 0.05 | 1 0.12 | 0.01 | 0.54 | 1.25 |
| NNN/pSbFucT | 0.10 | 0.87 | 1 0.09 | 0.01 | 0.11 | 1 0.27 |
| NNN/pPsFucT | 0.03 | 0.01 | 1 0.42 | 0.05 | 0.97 | 1.66 |
| NNN/pNbFucT2 | 0.28 | 1.60 | 1 0.11 | 0.01 | 0.09 | 1 0.34 |
| NNN/pCMfFucT | 0.04 | 0.02 | 1 0.53 | 0.06 | 1.02 | 1.54 |
| NNN/pWbwK | 0.03 | 0.01 | 1 0.72 | 0.08 | 1.14 | 2.16 |
| NNN/pWbiQ | 0.03 | N.D | 0.54 | 0.07 | 0.84 | 1.53 |
| NNN/pHMFT | 0.22 | 0.79 | 0.25 | 0.03 | 0.74 | 1.38 |
| NNN/pTe2FT | 0.02 | 0.05 | 1 0.18 | 0.02 | 0.66 | 1.13 |
| NNN/pFutC | 0.09 | 0.21 | 0.24 | 0.03 | 0.92 | 1.37 |
| NNN/pCtrl | N.D | N.D | 0.24 | 0.03 | 1.41 | 1.63 |

As a result, it was found that, as compared with an FutC or Te2FT expressing strain known to be capable of producing LNFPI using α1,2-fucosyltransferase, the NNN/pGsFucT strain, the NNN/pFsFucT strain, the NNN/pNbFucT1 strain, the NNN/pMtFucT strain, the NNN/pAjFucT strain, the NNN/pSbFucT strain, the NNN/pNbFucT2 strain, and the NNN/pHMFT strain were able to accumulate a large amount of LNFPI both in the supernatant and the bacterial cells.

Among them, FsFucT derived from Francisella sp. FSC1006 and NbFucT2 derived from Neisseriales bacterium, which showed significantly high productivity of LNFPI, were selected as candidates for α1,2-fucosyltransferase useful for LNFPI production.

### [Example 3] Preparation of LNFPI

The NNN/pFsFucT strain and NNN/pNbFucT2 strain selected in Example 2, and the NNN/pFucT54 strain constructed in Comparative Example as a positive control, were each cultured on an LB plate containing 100 mg/L kanamycin and 25 mg/L chloramphenicol at 30°C for 24 hours, and then inoculated into a 2 L baffled Erlenmeyer flask containing 250 mL of culture medium [yeast extract 5 g/L, peptone 10 g/L, sodium chloride 5 g/L] containing 100 mg/L kanamycin and 25 mg/L chloramphenicol, and cultured with shaking at 30°C for 17 hours.

Thereafter, 40 mL of the obtained culture solution was inoculated into a 3 L jar fermenter (manufactured by Mitsuwa Frontech Corp.) containing 760 mL of a production culture medium [glucose 20 g/L, ferrous sulfate heptahydrate 0.2 g/L, magnesium sulfate heptahydrate 2 g/L, disodium hydrogen phosphate 6 g/L, potassium dihydrogen phosphate 3 g/L, sodium chloride 5 g/L, ammonium chloride 1 g/L, yeast extract 5 g/L, manganese sulfate pentahydrate 10 mg/L, and thiamine hydrochloride 10 mg/L (aqueous solutions containing glucose, ferrous sulfate heptahydrate, and magnesium sulfate heptahydrate were prepared separately and then autoclaved, and each was cooled and then mixed)] containing 100 mg/L kanamycin and 25 mg/L chloramphenicol, and cultured with shaking at 30°C and 800 rpm for 72 hours. The pH was adjusted to 6.9 during the culture by adding 14% aqueous ammonia.

When the initial glucose was completely consumed, IPTG was added to a final concentration of 0.5 mM, and in the subsequent culture, a 480 g/L glucose solution and 4 g/L lactose monohydrate were added at a rate of 1 mL/h to 6 mL/h.

After completion of the culture, a culture solution was centrifuged and appropriately diluted, and LNFPI, 2'FL, LNTII, or LNT contained in a supernatant was analyzed using a carbohydrate analyzer ICS-5000. The results are shown in Table 7.

**[Table 7]**

| Strain name | LNFPI [g/L] | | 2'FL [g/L] | | LNTII [g/L] | | LNT [g/L] | |
|---|---|---|---|---|---|---|---|---|
| | Supernatant | Inside bacterial cells | Supernatant | Inside bacterial cells | Supernatant | Inside bacterial cells | Supernatant | Inside bacterial cells |
| NNN/pFucT54 | 6.7 | 3.1 | 4.1 | 0.6 | 0.5 | N.D | 0.7 | 0.3 |
| NNN/pFsFucT | 8.5 | 4.0 | 5.2 | 0.8 | 0.1 | N.D | 0.3 | 0.2 |
| NNN/pNbFucT2 | 14.5 | 4.9 | 1.2 | 0.4 | 0.2 | N.D | 0.3 | 0.2 |

As shown in Table 7, the NNN/pFsFucT strain and the NNN/pNbFucT2 strain produced the fucosylated oligosaccharides 2'FL and LNFPI, suggesting that FsFucT and NbFucT2 could be used for the production of oligosaccharides thereof. It was found that the NNN/pFsFucT strain and the NNN/pNbFucT2 strain accumulated significantly more LNFPI in both the supernatant and the bacterial cells as compared with the NNN/pFucT54 strain, which was a known α1,2-fucosyltransferase-expressing strain. Particularly, the NNN/pNbFucT2 strain produced approximately twice as much LNFPI as the NNN/pFucT54 strain. In addition, the NNN/pNbFucT2 strain showed significantly reduced production of the by-product 2'FL as compared with the other strains, suggesting that NbFucT2 may preferentially use LNT as a substrate.

Although the present invention has been described in detail with reference to specific embodiments, it is apparent to those skilled in the art that various changes and modifications can be made without departing from the spirit and scope of the present invention. The present application is based on a Japanese Patent Application No. 2022-050798 filed on March 25, 2022, the entire contents of which are incorporated herein by reference. All references cited herein are incorporated in their entirety.

### SEQUENCE LISTING FREE TEXT

SEQ ID NO: 1: nucleotide sequence of GsFucT derived from Gramella sp. MAR 2010 147
SEQ ID NO: 2: amino acid sequence of GsFucT derived from Gramella sp. MAR_2010_147
SEQ ID NO: 3: nucleotide sequence of FsFucT derived from Francisella sp. FSC1006
SEQ ID NO: 4: amino acid sequence of FsFucT derived from Francisella sp. FSC1006
SEQ ID NO: 5: nucleotide sequence of NbFucT1 derived from Neisseriaceae bacterium DSM 100970
SEQ ID NO: 6: amino acid sequence of NbFucT1 derived from Neisseriaceae bacterium DSM 100970
SEQ ID NO: 7: nucleotide sequence of MtFucT derived from Methylobacter tundripaludum
SEQ ID NO: 8: amino acid sequence of MtFucT derived from Methylobacter tundripaludum
SEQ ID NO: 9: nucleotide sequence of AjFucT derived from Amphritea japonica
SEQ ID NO: 10: amino acid sequence of AjFucT derived from Amphritea japonica
SEQ ID NO: 11: nucleotide sequence of PaFucT derived from Pseudohalocynthiibacter aestuariivivens
SEQ ID NO: 12: amino acid sequence of PaFucT derived from Pseudohalocynthiibacter aestuariivivens
SEQ ID NO: 13: nucleotide sequence of SbFucT derived from Sterolibacteriaceae bacterium J5B
SEQ ID NO: 14: amino acid sequence of SbFucT derived from Sterolibacteriaceae bacterium J5B
SEQ ID NO: 15: nucleotide sequence of PsFucT derived from Pedobacter sp. CF074
SEQ ID NO: 16: amino acid sequence of PsFucT derived from Pedobacter sp. CF074
SEQ ID NO: 17: nucleotide sequence of NbFucT2 derived from Neisseriales bacterium
SEQ ID NO: 18: amino acid sequence of NbFucT2 derived from Neisseriales bacterium
SEQ ID NO: 19: nucleotide sequence of CMfFucT derived from Candidatus Methylobacter favarea
SEQ ID NO: 20: amino acid sequence of CMfFucT derived from Candidatus Methylobacter favarea
SEQ ID NO: 21: nucleotide sequence of WbwK derived from Escherichia coli O86
SEQ ID NO: 22: amino acid sequence of WbwK derived from Escherichia coli O86
SEQ ID NO: 23: nucleotide sequence of wbiQ derived from Escherichia coli O127
SEQ ID NO: 24: amino acid sequence of wbiQ derived from Escherichia coli O127
SEQ ID NO: 25: nucleotide sequence of HMFT derived from Helicobacter mustelae ATCC 43772
SEQ ID NO: 26: amino acid sequence of HMFT derived from Helicobacter mustelae ATCC 43772
SEQ ID NO: 27: nucleotide sequence of FucT54 derived from Sideroxydans lithotrophicus ES-11
SEQ ID NO: 28: amino acid sequence of FucT54 derived from Sideroxydans lithotrophicus ES-11
SEQ ID NO: 29: nucleotide sequence of Te2FT derived from Thermosynechococcus elongates BP-1
SEQ ID NO: 30: amino acid sequence of Te2FT derived from Thermosynechococcus elongatus BP-1
SEQ ID NO: 31: nucleotide sequence of FutC derived from Helicobacter pylori 26695
SEQ ID NO: 32: amino acid sequence of FutC derived from Helicobacter pylori 26695
SEQ ID NO: 33: nucleotide sequence of Cvβ3galT derived from Chromobacterium violaceum ATCC 553
SEQ ID NO: 34: amino acid sequence of CvP3galT derived from Chromobacterium violaceum ATCC 553
SEQ ID NO: 35: nucleotide sequence of NplgtA derived from Neisseria polysaccharea ATCC 43768
SEQ ID NO: 36: amino acid sequence of NplgtA derived from Neisseria polysaccharea ATCC 43768
SEQ ID NO: 37: nucleotide sequences of catsacB fragment amplification primer
SEQ ID NO: 38: nucleotide sequences of catsacB fragment amplification primer
SEQ ID NO: 39: nucleotide sequence of lacZ upstream 1 amplification primer
SEQ ID NO: 40: nucleotide sequence of lacZ upstream 1 amplification primer
SEQ ID NO: 41: nucleotide sequence of lacY downstream 1 amplification primer
SEQ ID NO: 42: nucleotide sequence of lacY downstream 1 amplification primer
SEQ ID NO: 43: nucleotide sequence of lacZ upstream 2 amplification primer
SEQ ID NO: 44: nucleotide sequence of lacY downstream 2 amplification primer
SEQ ID NO: 45: nucleotide sequence of wcaJ upstream 1 amplification primer
SEQ ID NO: 46: nucleotide sequence of wcaJ upstream 1 amplification primer
SEQ ID NO: 47: nucleotide sequence of wcaM downstream 1 amplification primer
SEQ ID NO: 48: nucleotide sequence of wcaM downstream 1 amplification primer
SEQ ID NO: 49: nucleotide sequence of wcaJ upstream 2 amplification primer
SEQ ID NO: 50: nucleotide sequence of wcaM downstream 2 amplification primer
SEQ ID NO: 51: nucleotide sequence of Cvβ3galT fragment amplification primer
SEQ ID NO: 52: nucleotide sequence of Cvβ3galT fragment amplification primer
SEQ ID NO: 53: nucleotide sequences of NplgtA fragment amplification primer
SEQ ID NO: 54: nucleotide sequence of NplgtA fragment amplification primer
SEQ ID NO: 55: nucleotide sequence of lacY fragment amplification primer
SEQ ID NO: 56: nucleotide sequence of lacY fragment amplification primer
SEQ ID NO: 57: nucleotide sequence of pUAKQE fragment amplification primer
SEQ ID NO: 58: nucleotide sequence of pUAKQE fragment amplification primer
SEQ ID NO: 59: nucleotide sequence of rcsA fragment amplification primer
SEQ ID NO: 60: nucleotide sequence of rcsA fragment amplification primer
SEQ ID NO: 61: nucleotide sequence of pSTV29 amplification primer
SEQ ID NO: 62: nucleotide sequence of pSTV29 amplification primer
SEQ ID NO: 63: nucleotide sequence of GsFucT fragment amplification primer
SEQ ID NO: 64: nucleotide sequence of GsFucT fragment amplification primer
SEQ ID NO: 65: nucleotide sequence of FsFucT fragment amplification primer
SEQ ID NO: 66: nucleotide sequence of FsFucT fragment amplification primer
SEQ ID NO: 67: nucleotide sequence of NbFucT1 fragment amplification primer
SEQ ID NO: 68: nucleotide sequence of NbFucT1 fragment amplification primer
SEQ ID NO: 69: nucleotide sequence of MtFucT fragment amplification primer
SEQ ID NO: 70: nucleotide sequence of MtFucT fragment amplification primer
SEQ ID NO: 71: nucleotide sequence of AjFucT fragment amplification primer
SEQ ID NO: 72: nucleotide sequence of AjFucT fragment amplification primer
SEQ ID NO: 73: nucleotide sequence of PaFucT fragment amplification primer
SEQ ID NO: 74: nucleotide sequence of PaFucT fragment amplification primer
SEQ ID NO: 75: nucleotide sequence of SbFucT fragment amplification primer
SEQ ID NO: 76: nucleotide sequence of SbFucT fragment amplification primer
SEQ ID NO: 77: nucleotide sequence of PsFucT fragment amplification primer
SEQ ID NO: 78: nucleotide sequence of PsFucT fragment amplification primer
SEQ ID NO: 79: nucleotide sequence of NbFucT2 fragment amplification primer
SEQ ID NO: 80: nucleotide sequence of NbFucT2 fragment amplification primer
SEQ ID NO: 81: nucleotide sequences of CMfFucT fragment amplification primer
SEQ ID NO: 82: nucleotide sequences of CMfFucT fragment amplification primer
SEQ ID NO: 83: nucleotide sequence of WbwK amplification primer
SEQ ID NO: 84: nucleotide sequence of WbwK amplification primer
SEQ ID NO: 85: nucleotide sequence of WbiQ amplification primer
SEQ ID NO: 86: nucleotide sequence of WbiQ amplification primer
SEQ ID NO: 87: nucleotide sequence of HMFT fragment amplification primer
SEQ ID NOs: 88: nucleotide sequence of HMFT fragment amplification primer
SEQ ID NO: 89: nucleotide sequence of FucT54 fragment amplification primer
SEQ ID NO: 90: nucleotide sequence of FucT54 fragment amplification primer
SEQ ID NOs: 91: nucleotide sequence of Te2FT fragment amplification primer
SEQ ID NO: 92: nucleotide sequence of Te2FT fragment amplification primer
SEQ ID NO: 93: nucleotide sequence of FutC fragment amplification primer
SEQ ID NO: 94: nucleotide sequence of FutC fragment amplification primer
SEQ ID NO: 95: nucleotide sequence of pSTV-rcsA fragment amplification primer

## Claims

1. A protein according to any one of the following [1] to [3], which has a trans-fucosylation activity to lacto-N-tetraose (LNT):
[1] a protein consisting of the amino acid sequence represented by SEQ ID NO: 2, 4, 6, 8, 10, 14, 18, or 26,
[2] a mutant protein having an α1,2-fucosyltransferase activity and consisting of an amino acid sequence in which 1 to 20 amino acids are deleted, substituted, inserted, or added in the amino acid sequence represented by SEQ ID NO: 2, 4, 6, 8, 10, 14, 18, or 26, and
[3] a homologous protein having an α1,2-fucosyltransferase activity and consisting of an amino acid sequence having an identity of 90% or more with the amino acid sequence represented by SEQ ID NO: 2, 4, 6, 8, 10, 14, 18, or 26.

2. A DNA consisting of the nucleotide sequence represented by SEQ ID NO: 1, 3, 5, 7, 9, 13, 17, or 25 or a homologous sequence thereof and encoding the protein according to any one of [1] to [3] according to claim 1.

3. A recombinant DNA comprising the DNA according to claim 2.

4. A transformant obtained by transforming a host cell with the recombinant DNA according to claim 3.

5. The transformant according to claim 4, which is a microorganism with an enhanced activity of the protein of any one of [1] to [3] according to claim 1 and enhanced productivity of fucose-containing carbohydrate.

6. The transformant according to claim 5, wherein the microorganism is Escherichia coli.

7. A method for producing a fucose-containing carbohydrate, comprising: preparing the transformant according to any one of claims 4 to 6; and producing the fucose-containing carbohydrate in a culture using the transformant.

8. The production method according to claim 7, wherein the fucose-containing carbohydrate is lacto-N-fucopentaose I (LNFPI).
